# EUROPEAN PATENT APPLICATION

(11) **EP 3 739 328 A1**
(43) Date of publication of application: **18.11.2020**
(21) Application number: 18899287.9
(22) Date of filing: 09.01.2018
(51) Int. Cl.: G01N 27/62

(54) **METHOD FOR IDENTIFYING PROTEINS**

(71) Applicant: Shimadzu Corporation, Kyoto 604-8511 (JP)
(72) Inventor: SONOMURA, Kazuhiro, Kyoto-shi, Kyoto 604-8511 (JP); SATO, Taka-Aki, Kyoto-shi, Kyoto 604-8511 (JP); MATSUDA, Fumihiko, Kyoto-shi, Kyoto 606-8501 (JP); WANG Yi-Ting, Kyoto-shi, Kyoto 606-8501 (JP); ISHIHAMA, Yasushi, Kyoto-shi, Kyoto 606-8501 (JP); TSAI Chia-Feng, Kyoto-shi, Kyoto 606-8501 (JP)
(74) Representative: Kilian Kilian & Partner
(86) International application number: PCT/JP2018/000180
(87) International publication number: WO 2019/138441

(57) **Abstract**

Provided is a method for identifying proteins capable of increasing the number of identified proteins contained in a target sample derived from blood. A protein contained in the target sample derived from blood is fragmented, and a protein contained in an having less bias in a quantity ratio of proteins than the target sample is fragmented, and the fragmented proteins are mixed (Steps S101 to S103). In this manner, the mixed sample in which the bias in a quantity ratio of proteins is less than that of the target sample is generated. By performing MS/MS measurement using the generated mixed sample (Step S107), an MS/MS spectrum of a peak derived from a protein contained in a small amount in the target sample can be prevented from being missed. Accordingly, the number of identified proteins contained in the target sample derived from blood can be increased.

## Description

### TECHNICAL FIELD

The present invention relates to a method for identifying proteins for identifying a protein contained in a target sample derived from blood.

### BACKGROUND ART

In the field of proteome analysis, which performs comprehensive analysis of proteins, there is a case where each component in a sample is separated by liquid chromatography, and MS/MS measurement is sequentially performed on each of the separated components, so that each of the components is identified based on an obtained MS/MS spectrum (for example, see Patent Document 1 below). In this case, the protein sample is digested with a digestive enzyme such as trypsin, and a mixture of resulting peptide fragments (peptide mixture) is measured.

The above-described analysis method is known as a method called shotgun analysis using mass spectrometry. Specifically, each component separated by a liquid chromatograph is ionized, and mass spectrometry is performed on each of the ionized components, so that an MS spectrum is measured (MS measurement). Then, a peak with a high intensity is selected from data of the obtained MS spectrum, and an ion (precursor ion) corresponding to the peak is cleaved to perform mass spectrometry, so that an MS/MS spectrum is measured (MS/MS measurement). By performing a database search using the MS/MS spectrum obtained in this manner, identification of a peptide sequence and identification of a protein can be performed.

### PRIOR ART DOCUMENT

### PATENT DOCUMENT

Patent Document 1: Japanese Patent Laid-Open No. 2015-148461

### SUMMARY OF THE INVENTION

### PROBLEMS TO BE SOLVED BY THE INVENTION

In the above shotgun analysis, mass spectrometry is sequentially performed on each component separated and sent by liquid chromatography, and MS/MS measurement needs to be performed at relatively short time intervals (for example, about several seconds). For this reason, the number of ions that can be selected as precursor ions is limited, and it is difficult to perform MS/MS measurement by selecting peaks derived from all proteins contained in a sample.

In particular, in a case where there is a bias in a quantity ratio of a plurality of types of proteins contained in a sample, peaks derived from abundantly contained proteins are preferentially selected, and MS/MS measurement is performed using ions corresponding to those peaks as precursor ions. In this case, peaks derived from proteins contained in a small amount are less likely to be selected, and an MS/MS spectrum for ions corresponding to those peaks is not obtained in some cases.

For example, a sample derived from blood has a characteristic of being rich in some types of proteins (for example, albumin). In a case where shotgun analysis is performed on such a sample derived from blood, a peak derived from a protein contained in a large amount in the sample is preferentially selected and the MS/MS measurement is performed. Accordingly, there is a problem that an MS/MS spectrum for a peak derived from other proteins contained in a small amount is missed.

Therefore, a kit column (for example, "Multiple Affinity Removal Column Human 14" by Agilent Technologies) and the like for removing proteins contained in a large amount in a blood sample are also commercially available. However, even with such a kit column, the efficiency of protein removal is poor, or the concentration of proteins remaining after removal of a main protein is biased. Accordingly, the problem has not been completely solved in the present circumstances.

The present invention has been made in view of the above circumstances, and an object of the present invention is to provide a method for identifying proteins capable of increasing the number of identified proteins contained in a target sample derived from blood.

### MEANS FOR SOLVING THE PROBLEMS

The method for identifying proteins according to the present invention includes a first pretreatment step, a second pretreatment step, a mixing step, a separating step, an MS measurement step, an MS/MS measurement step, and an identifying step. In the first pretreatment step, fragmentation and labeling of a protein are performed on a target sample derived from blood, the target sample containing a plurality of types of proteins. In the second pretreatment step, fragmentation and labeling of a protein are performed on an additional sample containing a plurality of types of proteins with less bias in a quantity ratio of proteins than the target sample. In the mixing step, a mixed sample is generated by mixing the target sample after the first pretreatment step is performed with the additional sample that has undergone the second pretreatment. In the separating step, a component contained in the mixed sample is separated with a liquid chromatograph. In the MS measurement step, each of separated components from the mixed sample is ionized, and mass spectrometry is performed on each of the ionized components to measure an MS spectrum. In the MS/MS measurement step, an MS/MS spectrum is measured by cleaving a precursor ion selected based on the MS spectrum and performing mass spectrometry. In the identifying step, a protein contained in the target sample is identified based on the MS/MS spectrum.

According to such a configuration, a protein contained in the target sample derived from blood is fragmented, and a protein contained in the additional sample having less bias in a quantity ratio of proteins than the target sample is fragmented, and the fragmented proteins are mixed. In this manner, the mixed sample in which the bias in a quantity ratio of proteins is less than that of the target sample can be generated. Therefore, by performing MS/MS measurement using the generated mixed sample, an MS/MS spectrum of a peak derived from a protein contained in a small amount in the target sample can be prevented from being missed. Accordingly, the number of identified proteins in the target sample derived from blood can be increased.

Further, both the target sample derived from blood and the additional sample mixed with the target sample are mixed after labeling. Since a protein derived from the target sample and a protein derived from the additional sample can be clearly distinguished from each other in the above manner, only the protein contained in the target sample derived from blood can be reliably identified.

The additional sample may be a sample in which a quantity ratio of proteins is changed by applying a stimulus.

According to such a configuration, the additional sample in which a quantity ratio of proteins is changed by applying a stimulus is mixed with the target sample derived from blood, and MS/MS measurement is performed using the generated mixed sample, so that a protein increased in quantity by the stimulus can be identified.

The additional sample may be a sample derived from a cell or a tissue.

According to such a configuration, by using, as the additional sample, a sample derived from a cell or a tissue with less bias in a quantity ratio of proteins as compared with the target sample derived from blood, the number of identified proteins contained in the target sample derived from blood can be successfully increased.

### EFFECTS OF THE INVENTION

According to the present invention, a mixed sample in which a bias in a quantity ratio of proteins is reduced as compared with that of a target sample can be generated. Accordingly, by performing MS/MS measurement using the generated mixed sample, the number of identified proteins contained in the target sample derived from blood can be increased.

### BRIEF DESCRIPTION OF THE DRAWINGS

FIG. 1 is a block diagram showing a configuration example of an analyzer used when a method for identifying proteins according to one embodiment of the present invention is performed.
FIG. 2A is a diagram conceptually showing a relationship between a plurality of types of proteins contained in a target sample and an abundance.
FIG. 2B is a diagram conceptually showing a relationship between a plurality of types of proteins contained in an additional sample and an abundance.
FIG. 2C is a diagram conceptually showing a relationship between a plurality of types of proteins contained in a mixed sample and an abundance.
FIG. 3 is a flowchart showing an example of a method for identifying proteins according to one embodiment of the present invention.
FIG. 4A is a diagram showing a process of a procedure when a protein was identified using only plasma as a target sample.
FIG. 4B is a diagram showing a process of a procedure when a protein was identified by mixing a HeLa cell as an additional sample with plasma as a target sample.

### MODE FOR CARRYING OUT THE INVENTION

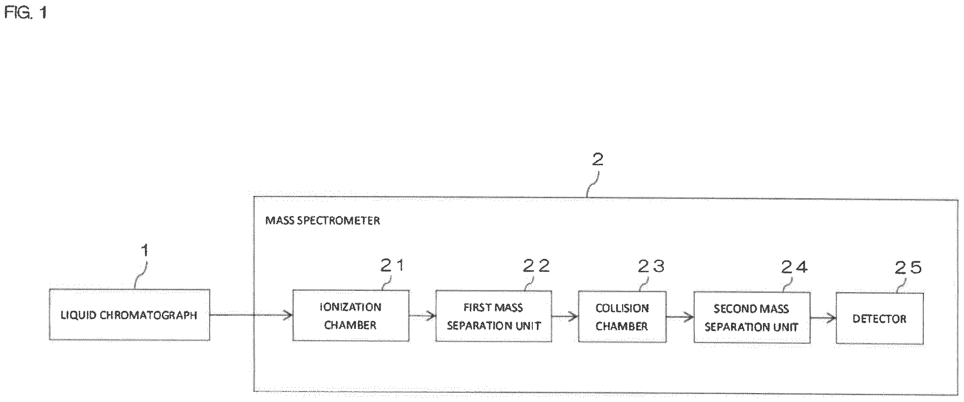

FIG. 1 is a block diagram showing a configuration example of an analyzer used when a method for identifying proteins according to one embodiment of the present invention is performed. This analyzer includes a liquid chromatograph 1 and a mass spectrometer 2.

The liquid chromatograph 1 includes a column (not shown), and components contained in a sample are temporally separated by the column. The components in the sample temporally separated in the liquid chromatograph 1 are sequentially sent to the mass spectrometer 2 with different holding times (retention times).

The mass spectrometer 2 includes an ionization chamber 21, a first mass separation unit 22, a collision chamber 23, a second mass separation unit 24, a detector 25, and the like. The components in the sample separated in the liquid chromatograph 1 are sequentially sent to the ionization chamber 21, where the components are ionized. The components in the sample can be ionized using a publicly-known ionization method such as, for example, electrospray ionization (ESI) or atmospheric pressure chemical ionization (APCI).

The first mass separation unit 22 includes, for example, a quadrupole mass filter, and selects and allows only ions having a predetermined mass-to-charge ratio to pass through. The ions that have passed through the first mass separation unit 22 are sent to the second mass separation unit 24 via the collision chamber 23. In the collision chamber 23, the ions from the first mass separation unit 22 are caused to collide with gas (argon, nitrogen, and the like) and cleaved, so that fragmented product ions can be generated.

The second mass separation unit 24 includes, for example, a quadrupole mass filter, like the first mass separation unit 22, and can allow only ions having a predetermined mass-to-charge ratio to pass through. The ions that have passed through the second mass separation unit 22 are detected by the detector 25, and the detector 25 outputs a detection signal according to an amount of the detected ions. Based on the detection signal from the detector 25, spectrum data can be obtained.

However, the first mass separation unit 22 and the second mass separation unit 24 are not limited to the configuration including the quadrupole mass filter. For example, a configuration using an ion trap as the first mass separation unit 22 or a configuration using a time-of-flight mass spectrometer (TOF-MS) as the second mass separation unit 24 can also be employed.

In this analyzer, components in a sample ionized in the ionization chamber 21 are detected by the detector 25 without being cleaved in the collision chamber 23 and mass spectrometry is performed, so that an MS spectrum can be measured (MS measurement). Further, a peak with a high intensity is selected from data of an MS spectrum, and an ion (precursor ion) corresponding to the peak is cleaved in the collision chamber 23 to generate a product ion. The product ion is detected by the detector 25 and mass spectrometry is performed, so that an MS/MS spectrum can be measured (MS/MS measurement).

In the present embodiment, a sample derived from blood, the target sample containing a plurality of types of proteins is set as a target to be measured (target sample), and a mixed sample obtained by mixing an additional sample with the target sample is prepared. The mixed sample is measured by the above-described analyzer, so that the proteins contained in the target sample are identified. The additional sample is not particularly limited as long as the additional sample is a sample containing a plurality of types of proteins in which a quantity ratio of the proteins is less biased than the target sample. For example, a sample derived from a cell or a tissue is preferably used. Note that, as the sample derived from blood, for example, plasma, serum, whole blood or blood cells can be exemplified. Further, the sample derived from a cell is a sample derived from various cells such as a cancer cell, and the sample derived from a tissue is a sample derived from a tissue made of various cells.

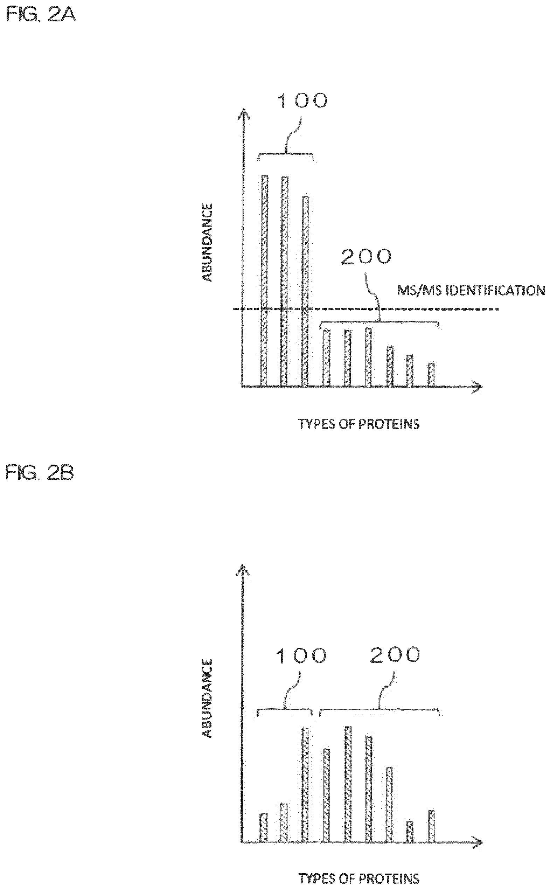

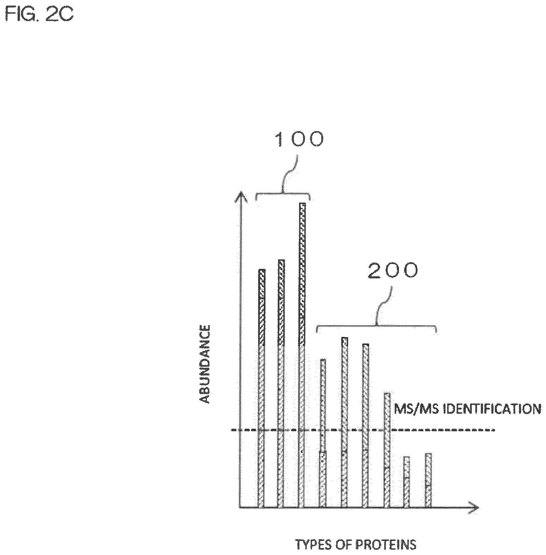

FIG. 2A is a diagram conceptually showing a relationship between a plurality of types of proteins contained in the target sample and an abundance. FIG. 2B is a diagram conceptually showing a relationship between a plurality of types of proteins contained in the additional sample and an abundance. FIG. 2C is a diagram conceptually showing a relationship between a plurality of types of proteins contained in the mixed sample and an abundance.

As shown in FIG. 2A, in the target sample derived from blood, there is a large bias in a quantity ratio of a plurality of types of proteins contained in the target sample. That is, there is a relatively large difference in abundance between protein types 100 having a large abundance and other protein types 200. Therefore, a peak corresponding to the protein types 100 having a large abundance is easily detected with high intensity. As a result, peaks corresponding to the other protein types 200 are less likely to be selected as a precursor ion, and an MS/MS spectrum of ions corresponding to the peaks cannot be obtained in some cases. In this case, the protein types 200 having a small abundance cannot be identified based on an MS/MS spectrum in some cases even though the protein types are included in the target sample.

On the other hand, as shown in FIG. 2B, in the additional sample derived from a cell or a tissue, the bias in a quantity ratio of a plurality of types of proteins contained in the additional sample is smaller than that of the target sample shown in FIG. 2A. That is, a difference in abundance between the protein types having a large abundance and the protein types having a small abundance is not as large as that of the target sample. The protein types contained in the additional sample and the protein types contained in the target sample overlap at least partially. In the examples of FIGS. 2A and 2B, while both the protein types 100 having a large abundance in the target sample and the other protein types 200 are contained in the additional sample, the bias in the quantity ratio of these is smaller than that of the target sample.

In a case where the target sample and the additional sample as described above are mixed, as shown in FIG.2C, the bias in the quantity ratio of a plurality of types of proteins contained in the obtained mixed sample is smaller than that in the case of the target sample alone shown in FIG.2A. That is, not only the protein types 100 that has a large abundance in the case of the target sample alone, but also the abundance of at least part of the other protein types 200 is large. As a result, the bias in the quantity ratio of proteins is smaller than that in the case of the target sample alone.

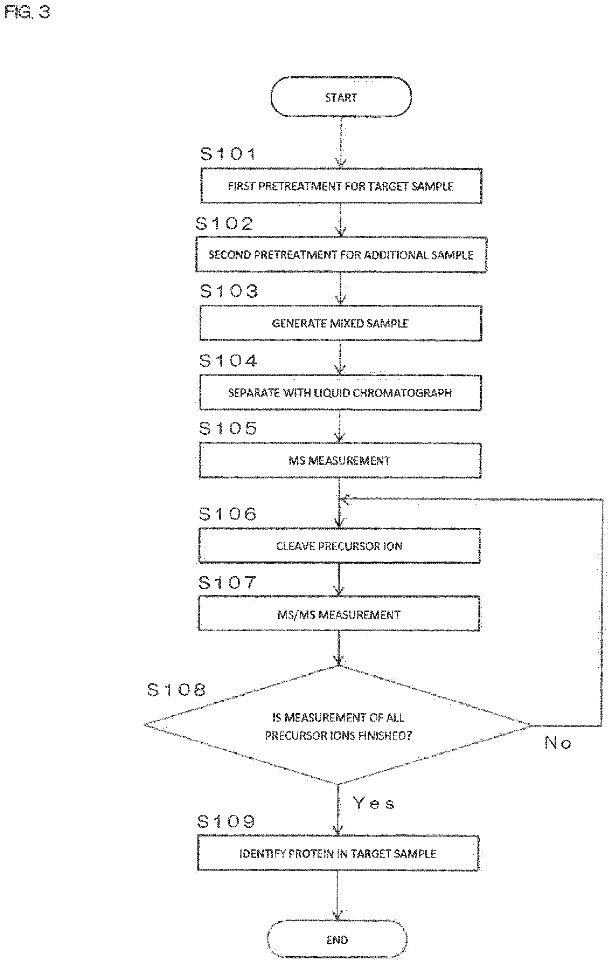

FIG. 3 is a flowchart showing an example of a method for identifying proteins according to one embodiment of the present invention. When a protein contained in the target sample is identified, first, a first pretreatment is performed on the target sample (Step S101: first pretreatment step), and a second pretreatment is performed on the additional sample (Step S102: second pretreatment step). Then, the target sample and the additional sample are mixed to generate a mixed sample (Step S103: mixing step).

In the first pretreatment step (Step S101), protein fragmentation and labeling are performed on the target sample. Specifically, a protein contained in the target sample is digested by a digestive enzyme such as trypsin, and fragmented into peptide fragments. Further, labeling is performed by label-modifying a protein contained in the target sample using a stable isotope reagent such as a TMT reagent manufactured by Thermo Fisher Scientific Inc. Since a method of fragmentation and labeling is well known, detailed description of the method will be omitted. Note that either one of fragmentation and labeling of a protein on the target sample may be performed first.

In the second pretreatment step (Step S102), fragmentation and labeling of a protein are performed on the additional sample. The method of fragmentation and labeling of a protein on the additional sample is similar to that of the first pretreatment step on the target sample, and either one of fragmentation and labeling may be performed first. Further, either one of the first pretreatment step and the second pretreatment step may be performed first, or the steps may be performed in parallel. The labeling of a protein is preferably performed using different reagents that cause a mass difference between the target sample and the additional sample when a precursor ion is cleaved during MS/MS measurement.

The target sample after the first pretreatment step is performed is mixed with the additional sample after the second pretreatment step to form a mixed sample (Step S103), and the mixed sample is introduced into the liquid chromatograph 1. In this manner, components contained in the mixed sample are separated by the liquid chromatograph 1 (Step S104: separating step).

Each of the components from the mixed sample separated by the liquid chromatograph 1 is introduced into the mass spectrometer 2. In the mass spectrometer 2, first, each of the separated components from the mixed sample is ionized in the ionization chamber 21, and mass spectrometry for each of the ionized components is performed, so that an MS spectrum is measured (Step S105: MS measurement step).

After the above, a peak whose intensity is equal to or larger than a threshold is selected from data of the MS spectrum, so that an ion corresponding to the peak is selected as a precursor ion. Then, the selected precursor ion is cleaved in the collision chamber 23, so that a product ion is generated. Mass spectrometry is performed on the product ion, so that an MS/MS spectrum is measured (Steps S106 and S107: MS/MS measurement step).

The MS/MS measurement steps (Steps S106 and S107) as described above are performed for all the selected precursor ions. Then, after the MS/MS measurement step is performed on all the precursor ions (Yes in Step S108), a protein contained in the target sample is identified based on the obtained MS/MS spectrum (Step S109: identifying step). This identifying step can be performed using a publicly-known method such as a database search.

As described above, in the present embodiment, after the target sample and the additional sample are mixed to generate the mixed sample (Steps S101 to S103), the LC-MS/MS analysis (Steps S104 to S109) on the mixed sample is performed. That is, a protein contained in the target sample derived from blood is fragmented, and a protein contained in the additional sample having less bias in a quantity ratio of proteins than the target sample is fragmented, and the fragmented proteins are mixed. In this manner, as shown in FIG. 2C, the mixed sample in which the bias in a quantity ratio of proteins is less than that of the target sample can be generated. Therefore, by performing MS/MS measurement using the generated mixed sample, an MS/MS spectrum of a peak derived from a protein contained in a small amount in the target sample can be prevented from being missed. Accordingly, the number of identified proteins in the target sample derived from blood can be increased.

Further, both the target sample derived from blood and the additional sample mixed with the target sample are mixed after the labeling is performed in the first pretreatment step (Step S101) or the second pretreatment step (Step S102) (Step S103). Since a protein derived from the target sample and a protein derived from the additional sample can be clearly distinguished from each other in the above manner, only the protein contained in the target sample derived from blood can be reliably identified.

In particular, when a sample derived from a cell or a tissue with less bias in a quantity ratio of proteins as compared with the target sample derived from blood is used as the additional sample, the bias in a quantity ratio of proteins can be successfully reduced as shown in FIG. 2C. Therefore, the number of identified proteins contained in the target sample derived from blood can be successfully increased.

Instead of simply mixing the target sample and the additional sample as described above, the target sample can be mixed after a stimulus is applied to the additional sample. Examples of a method of applying a stimulus to the additional sample include various methods such as heating or cooling or stirring the additional sample, in addition to a method of adding an agent to the additional sample. By applying a stimulus to the additional sample as described above, the additional sample is converted into a sample in which a quantity ratio of proteins is changed, and then mixed with the target sample. By performing the MS/MS measurement using the mixed sample thus generated, a protein increased in quantity by the stimulus can be identified.

Hereinafter, a result of an effect test in which the number of identified proteins contained in the target sample can be increased by mixing the additional sample with the target sample derived from blood and performing LC-MS/MS analysis will be described. Plasma was used as the target sample derived from blood, and a HeLa cell, which was a sample derived from a cell, was used as the additional sample. Further, TMT reagents (TMT126 and TMT131) manufactured by Thermo Fisher Scientific Inc. were used for labeling proteins, and Orbitrap-Fusion manufactured by the same company was used for measurement. Note that, plasma is a typical sample containing a large amount of some proteins such as albumin and having a bias in a quantity ratio of the proteins.

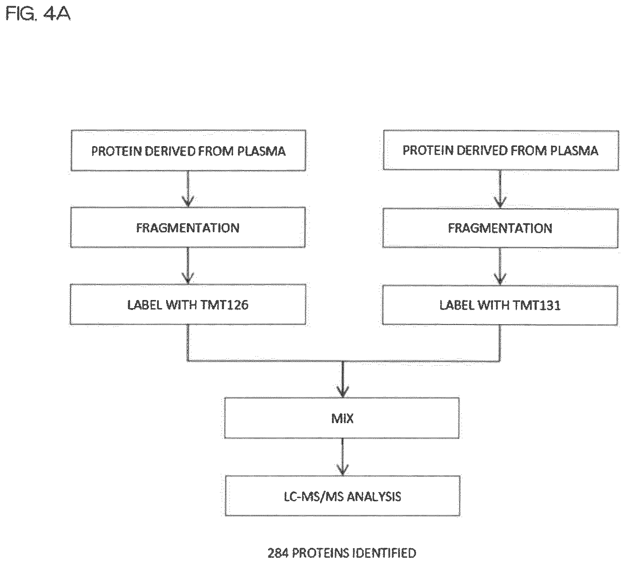

FIG. 4A is a diagram showing a process of a procedure when a protein was identified using only plasma as the target sample. In this case, the target sample obtained by fragmenting a protein derived from plasma and labeling the protein with TMT126 was mixed with the target sample obtained by fragmenting a protein derived from plasma and labeling the protein with TMT131, and LC-MS/MS analysis was performed on the mixed sample. As a result, the number of identified proteins in the target sample was "284".

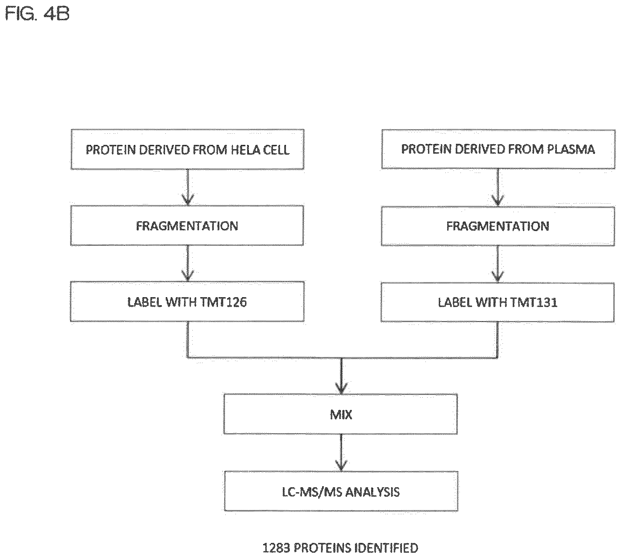

FIG. 4B is a diagram showing a process of a procedure when a protein was identified by mixing a HeLa cell as the additional sample with plasma as the target sample. In this case, the additional sample obtained by fragmenting a protein derived from a HeLa cell and fragmenting the protein with TMT126 was mixed with the target sample obtained by fragmenting a protein derived from plasma and labeling the protein with TMT131, and LC-MS/MS analysis was performed on the mixed sample. As a result, the number of identified proteins contained in the target sample was "1283", and the number of identified proteins was dramatically increased.

### DESCRIPTION OF REFERENCE SIGNS

- 1: liquid chromatograph
- 2: mass spectrometer
- 21: ionization chamber
- 22: mass separation unit
- 23: collision chamber
- 24: mass separation unit
- 25: detector

## Claims

1. A method for identifying proteins, comprising:
a first pretreatment step of performing fragmentation and labeling on a protein for a target sample derived from blood, the target sample containing a plurality of types of proteins;
a second pretreatment step of performing fragmentation and labeling on a protein for an additional sample containing a plurality of types of proteins with less bias in a quantity ratio of proteins than the target sample;
a mixing step of generating a mixed sample by mixing the additional sample that has undergone the second pretreatment with the target sample after the first pretreatment step is performed;
a separating step of separating the mixed sample into components with a liquid chromatograph;
an MS measurement step of measuring an MS spectrum by ionizing each of separated components from the mixed sample and performing mass spectrometry for each of the ionized components;
an MS/MS measurement step of measuring an MS/MS spectrum by performing mass spectrometry by cleaving a precursor ion selected based on the MS spectrum; and
an identifying step of identifying a protein contained in the target sample based on the MS/MS spectrum.

2. The method for identifying proteins according to claim 1, wherein the additional sample is a sample in which a quantity ratio of proteins is changed by applying a stimulus.

3. The method for identifying proteins according to claim 1 or 2, wherein the additional sample is a sample derived from a cell or a tissue.
